# EUROPEAN PATENT SPECIFICATION

(11) **EP 4 021 291 B1**
(45) Date of publication and mention of the grant of the patent: **26.11.2025**
(21) Application number: 20756869.2
(22) Date of filing: 19.08.2020
(51) Int. Cl.: A61B 5/055, A61B 5/00

(54) **SYSTEM FOR FUNCTIONAL MAGNETIC RESONANCE IMAGE DATA ACQUISITION**
SYSTEM ZUR FUNKTIONELLEN MAGNETRESONANZBILDDATENERFASSUNG MIT INTEGRIERTEM EEG
SYSTÈME D'ACQUISITION DE DONNÉES D'IMAGE PAR RÉSONANCE MAGNÉTIQUE FONCTIONNELLE AVEC EEG

(30) Priority: 29.08.2019 EP 19194341
(43) Date of publication of application: 06.07.2022
(73) Proprietor: Koninklijke Philips N.V., 5656 AG Eindhoven (NL)
(72) Inventor: FLÄSCHNER, Nick, 5656 AE Eindhoven (NL); EWALD, Arne, 5656 AE Eindhoven (NL); LAMERICHS, Rudolf, Mathias, Johannes, Nicolaas, 5656 AE Eindhoven (NL)
(74) Representative: Philips Intellectual Property & Standards
(86) International application number: PCT/EP2020/073150
(87) International publication number: WO 2021/037617

(56) References cited:
- US-A1- 2013 267 827
- SILVINA G. HOROVITZ ET AL: "Low frequency BOLD fluctuations during resting wakefulness and light sleep: A simultaneous EEG-fMRI study", HUMAN BRAIN MAPPING, vol. 29, no. 6, 1 June 2008 (2008-06-01), pages 671 - 682, XP055665217, ISSN: 1065-9471, DOI: 10.1002/hbm.20428

## Description

### FIELD OF THE INVENTION

The present invention relates to system and method for functional magnetic resonance image data acquisition.

### BACKGROUND OF THE INVENTION

Functional magnetic resonance imaging (fMRI) measures brain activity through detecting blood flow, and where blood flow in the brain and spinal column and brain activity are linked. A blood-oxygen-level dependent (BOLD) contrast signal is frequently used, where neural activity in the brain or spinal cord of humans or other animals is mapped by imaging the change in blood flow (hemodynamic response) related to energy use by brain cells. fMRI has advantages in that the patient does not have to undergo surgery or be exposed to a radiation dose. However, the technique does suffer from the effects of noise.

S.G. Horovitz et al., Hum Brain Mapp 29:671-682 2008 describes that recent blood oxygenation level dependent functional MRI (BOLD fMRI) studies of the human brain have shown that in the absence of external stimuli, activity persists in the form of distinct patterns of temporally correlated signal fluctuations. In this work, the authors investigated the spontaneous BOLD signal fluctuations during states of reduced consciousness such as drowsiness and sleep. For this purpose, they performed BOLD fMRI on normal subjects during varying levels of consciousness, from resting wakefulness to light (non-slow wave) sleep. Depth of sleep was determined based on concurrently acquired EEG data. During light sleep, significant increases in the fluctuation level of the BOLD signal were observed in several cortical areas, among which visual cortex was the most significant. Correlations among brain regions involved with the default-mode network persisted during light sleep. The authors described that these results suggest that activity in areas such as the default-mode network and primary sensory cortex, as measured from BOLD fMRI fluctuations, does not require a level of consciousness typical of wakefulness. US 2013/267827 discloses a combined EEG / MRI system which - when no alpha waves or predominantly delta waves are detected - warns the operator or plays a noise via headphones worn by the patient.

fMRI can be used to monitor neuronal activity during or whilst are being performed by the patient, for example when tasks have been given to the patient to do. However, for the fMRI data to be representative the patient must be compliant with the test or task.

There is a need to address these issues.

### SUMMARY OF THE INVENTION

It would be advantageous to have improved techniques for functional magnetic resonance image data acquisition.

The object of the present invention is solved with the subject matter of the independent claims, wherein further embodiments are incorporated in the dependent claims. It should be noted that the following described aspects and examples of the invention apply both to the system for functional magnetic resonance image data acquisition, and to the method for functional magnetic resonance image data acquisition.

In a first aspect, there is provided a system for functional magnetic resonance image data acquisition, the system comprising:
- an input unit;
- a magnetic resonance imaging "MRI" device;
- an electroencephalography "EEG" data acquisition device; and
- a processing unit.

The input unit is configured to provide task-based information to a patient, wherein the task-based information extends over a period of time, and wherein the information relating to one or more tasks relates to a visual task and/or an auditory task and/or a motor task and/or a task to enter a resting state and/or a task to fall asleep and/or a task to stay awake. The MRI device is configured to acquire functional magnetic resonance imaging "fMRI" data relating to brain activity of the patient. The fMRI data extends over the period of time. The EEG device is configured to acquire EEG data relating to electrical activity of the brain of the patient. The EEG data extends over the period of time. The processing unit is configured to utilize the task-based information that extends over the period of time and the EEG data that extends over the period of time to determine at least one first sub-set period of time over the period of time, wherein the processing unit is configured to analyze the EEG data in real-time to determine if the patient is compliant with at least one task. The processing unit is configured to determine an action associated with acquisition of the fMRI data over the at least one first sub-set period of time.

In other words, an EEG device that is fMRI compatible is utilized to provide real-time data that can be easily analysed in real-time to determine if a patient is compliant with a task. This enables an identification of the fMRI data being acquired when the patient is not in compliance with task enabling that data over a specific time period to marked with respect to fMRI analysis and also enabling the patient to be prompted to continue correctly with the task. Also, the validly acquired fMRI data, when the patient is in compliance with the task can be identified enabling this fMRI data over specific time periods of validity to be further analysed. The fMRI data marked for the period of time when the patient was not in compliance could then be discarded. However, the fMRI data itself during the period of non-compliance can be compared to that when the patient was compliant.

In this manner, an improved task-based fMRI system is provided.

The task-based information provided to the patient can for example relate to visual task such as watching a video or looking at pictures or photographs, auditory tasks with or without video, or tasks such as requesting to tap fingers or carry out other motor tasks.

The task-based information can be a request for the patient to become restful or enter a resting state and even try to fall asleep, and the task-based information can be a request to be active, and attentive and not fall asleep. Thus, compliance with the task can be to be in a resting state or to be in an active state.

The determined action comprises a provision of a prompt that the patient is not in compliance with at least one task over the at least one first sub-set period of time.

The prompt is provided to the patient and/or an operator of the system that the patient is not in compliance with at least one task over the at least one first sub-set period of time

The determined action comprises an identification of the associated fMRI data over the first sub-set period of time as data when the patient was not in compliance with at least one task.

In an example, the processing unit is configured to utilize the task-based information and the EEG data over the period of time to determine at least one second sub-set period of time over the period of time. The processing unit is configured to determine an identification of the associated fMRI data over the second sub-set period of time as data when the patient was in compliance with at least one task over the at least one second sub-set period of time.

In an example, the input unit comprises a visual display unit "VDU" and wherein provision of task-based information comprises a display of a video or photograph on the VDU that the patient is requested to watch or look at.

In an example, the utilization of the task-based information and the EEG data by the processing unit comprises a determination that the patient is watching the video or looking at the photograph.

In an example, the determination that the patient is watching the video or looking at the photograph comprises an analysis of the EEG data to determine a level of visual attentiveness of the patient.

In an example, the determination that the patient is watching the video or looking at the photograph comprises an analysis of the EEG data to an alpha power at the visual cortex.

In a second aspect, there is provided a method for functional magnetic resonance image data acquisition, the method comprising:
a) providing by an input unit task-based information to a patient, the task-based information extending over a period of time, wherein the information relating to one or more tasks relates to a visual task and/or an auditory task and/or a motor task and/or a task to enter a resting state and/or a task to fall asleep and/or a task to stay awake;
b) acquiring by a magnetic resonance imaging "MRI" device functional magnetic resonance imaging "fMRI" data relating to brain activity of the patient, the fMRI data extending over the period of time;
c) acquiring by an electroencephalography "EEG" device EEG data relating to electrical activity of the brain of the patient, the EEG data extending over the period of time;
d) utilizing by a processing unit the task-based information and the EEG data and determining at least one first sub-set period of time over the period of time, and analyzing the EEG data in real-time to determine if the patient is compliant with at least one task; and
f) determining by the processing unit an action associated with acquisition of the fMRI data over the at least one first sub-set period of time.

Step f) comprises providing a prompt that the patient is not in compliance with at least one task over the at least one first sub-set period of time.

Step f) comprises identifying the associated fMRI data over the first sub-set period of time as data when the patient was not in compliance with at least one task.

In an example, the method comprises step e) utilizing by the processing unit the task-based information and the EEG data over the period of time to determine at least one second sub-set period of time over the period of time; and the method comprises step g) identifying by the processing unit the associated fMRI data over the second sub-set period of time as data when the patient was in compliance at least one task over the at least one second sub-set period of time.

In an example, there is provided a system for functional magnetic resonance image data acquisition, the system comprising:
- a magnetic resonance imaging "MRI" device;
- an electroencephalography "EEG" data acquisition device; and
- a processing unit.

The MRI device is configured to acquire functional magnetic resonance imaging "fMRI" data relating to brain activity of the patient. The fMRI data extends over the period of time. The EEG device is configured to acquire EEG data relating to electrical activity of the brain of the patient. The EEG data extends over the period of time. The processing unit is configured to utilize the EEG data that extends over the period of time to determine at least one sub-set period of time over the period of time associated with a level of wakefulness of the patient. The processing unit is configured to determine an action associated with acquisition of the fMRI data over the at least one sub-set period of time.

Thus, fMRI data during a resting state can be identified and fMRI data in an active state can be identified. Then, both sets of data can be utilized, where in an specific example 5 minutes of each state is required for further analysis. It is to be noted, that the system can also monitor resting and active states and select or identify the fMRI data that extends over time periods of each of these states in addition to doing so for different states of wakefulness.

In an example, the determined action comprises an identification of the associated fMRI data over the sub-set period of time as data when the patient was asleep or an identification of the associated fMRI data over the sub-set period of time as data when the patient was awake.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a system for functional magnetic resonance image data acquisition;
Fig. 2 shows a method for functional magnetic resonance image data acquisition; and
Fig. 3 shows a schematic set up of an example of a system for functional magnetic resonance image data acquisition.

### DETAILED DESCRIPTION OF EMBODIMENTS

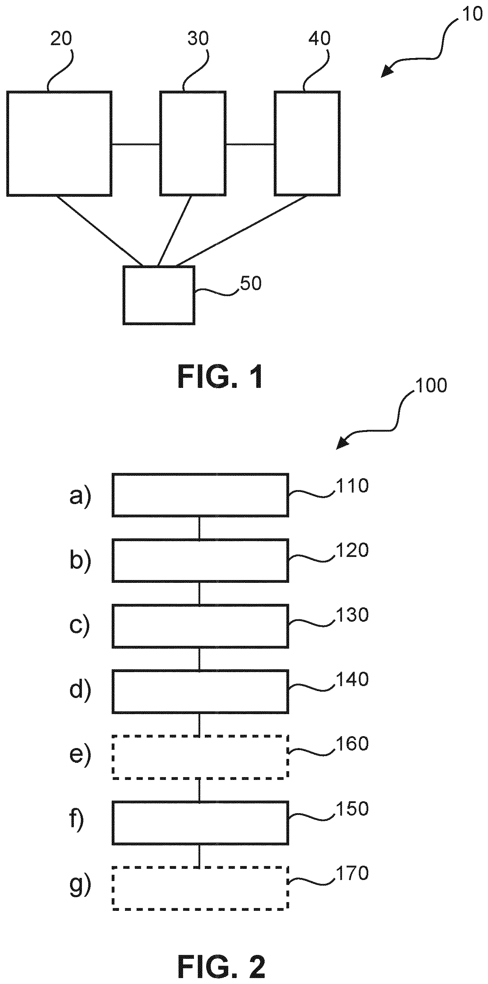

Fig. 1 shows an example of a system 10 for functional magnetic resonance image data acquisition. The system 10 comprises an input unit 20, a magnetic resonance imaging "MRI" device 30, an electroencephalography "EEG" data acquisition device 40, and a processing unit 50. The input unit is configured to provide task-based information to a patient. The task-based information extends over a period of time. The MRI device is configured to acquire functional magnetic resonance imaging "fMRI" data relating to brain activity of the patient. The fMRI data extends over the period of time. The EEG device is configured to acquire EEG data relating to electrical activity of the brain of the patient. The EEG data extends over the period of time. The processing unit is configured to utilize the task-based information that extends over the period of time and the EEG data that extends over the period of time to determine at least one first sub-set period of time over the period of time. The processing unit is configured also to determine an action associated with acquisition of the fMRI data over the at least one first sub-set period of time.

The determined action comprises a provision of a prompt that the patient is not in compliance with at least one task over the at least one first sub-set period of time.

The prompt is provided to the patient and/or an operator of the system that the patient is not in compliance with at least one task over the at least one first sub-set period of time

In an example, the input unit that provides the task-based information to the patient also provides the prompt to the patient. Thus, for example a visual display unit can provide video information to the patient to the watch and the VDU can flash and/or provide sound information to the patient to continue to watch the video.

In an example, the input unit that provides the task-based information to the patient is different a unit that provides the prompt to the patient and/or the operator. Thus, for example a visual display unit can provide video information to the patient to watch and a buzzer can sound to indicate that the patient should continue to watch the video. Other mechanisms by which the patient and/or operator can be notified that the patient should continue with a task can also be provided.

The determined action comprises an identification of the associated fMRI data over the first sub-set period of time as data when the patient was not in compliance with at least one task.

According to an example, the processing unit is configured to utilize the task-based information and the EEG data over the period of time to determine at least one second sub-set period of time over the period of time. The processing unit is configured to determine an identification of the associated fMRI data over the second sub-set period of time as data when the patient was in compliance with at least one task over the at least one second sub-set period of time.

According to an example, the input unit comprises a visual display unit "VDU" and wherein provision of task-based information comprises a display of a video or photograph on the VDU that the patient is requested to watch or look at.

In an example, the patient is requested to view a video or look at a photograph for a set period of time, for example one minute.
In an example, the patient is requested to look at a drawing or schematic drawing other visual form for a set period of time, for example one minute.

In an example, the video, photograph or drawing or visual form can have a representation indicating happiness, such as a smile or can have a representation indicating sadness, in an appropriate manner.

In an example, the patient is requested to view one video for 1 minute and then view a second video for 1 minute.

In an example, the patient is requested to look at one photograph for 1 minute and then look at a second photograph for 1 minute.

According to an example, the utilization of the task-based information and the EEG data by the processing unit comprises a determination that the patient is watching the video or looking at the photograph.

According to an example, the determination that the patient is watching the video or looking at the photograph comprises an analysis of the EEG data to determine a level of visual attentiveness of the patient.

According to an example, the determination that the patient is watching the video or looking at the photograph comprises an analysis of the EEG data to an alpha power at the visual cortex.

Fig. 2 shows an example of a method 100 for functional magnetic resonance image data acquisition in its basic steps where essential steps are shown in solid lines and optional steps are shown in dashed lines. The method comprises:
- in a providing step 110, also referred to as step a), providing by an input unit task-based information to a patient, the task-based information extending over a period of time;
- in an acquiring step 120, also referred to as step b), acquiring by a magnetic resonance imaging "MRI" device functional magnetic resonance imaging "fMRI" data relating to brain activity of the patient, the fMRI data extending over the period of time;
- in an acquiring step 130, also referred to as step c), acquiring by an electroencephalography "EEG" device EEG data relating to electrical activity of the brain of the patient, the EEG data extending over the period of time;
- in a utilizing step 140, also referred to as step d), utilizing by a processing unit the task-based information and the EEG data and determining at least one first sub-set period of time over the period of time; and
- in a determining step 150, also referred to as step f), determining by the processing unit determine an action associated with acquisition of the fMRI data over the at least one first sub-set period of time.

Step f) comprises providing a prompt that the patient is not in compliance with at least one task over the at least one first sub-set period of time.

Step f) comprises providing the prompt to the patient and/or an operator of the system that the patient is not in compliance with at least one task over the at least one first sub-set period of time

Step f) comprises identifying the associated fMRI data over the first sub-set period of time as data when the patient was not in compliance with at least one task.

According to an example, the method comprises step e) utilizing 160 by the processing unit the task-based information and the EEG data over the period of time to determine at least one second sub-set period of time over the period of time; and the method comprises step g) identifying 170 by the processing unit the associated fMRI data over the second sub-set period of time as data when the patient was in compliance at least one task over the at least one second sub-set period of time.

In an example, step a) comprises displaying a video on a visual display unit "VDU" that the patient is requested to watch.

In an example, step d) comprises determining that the patient is watching the video.

In an example, step d) comprises analysing the EEG data and determining a level of visual attentiveness of the patient.

In an example, determining the level of visual attentiveness of the patient comprises analyzing the EEG data and determining an alpha power at the visual cortex.

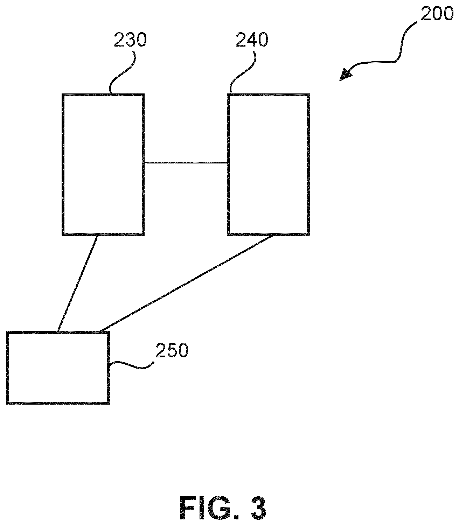

Fig. 3 shows an example of a system 200 for functional magnetic resonance image data acquisition. The system 200 comprises a magnetic resonance imaging "MRI" device 230, an electroencephalography "EEG" data acquisition device 240, and a processing unit 250. The MRI device is configured to acquire functional magnetic resonance imaging "fMRI" data relating to brain activity of the patient. The fMRI data extends over the period of time. The EEG device is configured to acquire EEG data relating to electrical activity of the brain of the patient. The EEG data extends over the period of time. The processing unit is configured to utilize the EEG data that extends over the period of time to determine at least one sub-set period of time over the period of time associated with a level of wakefulness of the patient. The processing unit is configured also to determine an action associated with acquisition of the fMRI data over the at least one sub-set period of time.

According to an example, the determined action comprises an identification of the associated fMRI data over the sub-set period of time as data when the patient was asleep or an identification of the associated fMRI data over the sub-set period of time as data when the patient was awake.

Thus, the described system and method for functional magnetic resonance image data (fMRI) acquisition with respect to task-based fMRI and/or resting state fMRI uses acquired EEG data in a completely new way in order to determine when the fMRI data is valid or not (whether for example a patient is carrying out a task). The EEG data over a time window is correlated with task information over that time window to determine if the patient is carrying out the task (or is asleep or awake). Then if the patient, from analysis of the EEG data, is in compliance over that time window the associated fMRI data over that time window can be analysed with respect to the task being carried out at the same time. If the patient, over that time window, is determined from the EEG data not to be compliant, the fMRI data over that time window can be disregarded and the patient prompted to continue with the task. Similarly, when fMRI data is required for a patient who is asleep (or awake) the EEG data over a time window can be analysed to determine if the patient is asleep or awake over that time window and the fMRI data over that time window can be utilized or not as the case requires.

To put this another way, it is not possible easily to monitor patient compliance during (task-based or resting-state) fMRI because, real-time analysis of the BOLD signal suffers from a small signal-to-noise ratio and a low temporal resolution. Thus, it is often found only after the fMRI acquisition that the data cannot be used or can only partially be used, requiring an expensive and time consuming repeat fMRI scan The new technique described here overcomes this lack of patient compliance for fMRI data by monitoring patient compliance in real time and providing feedback to the practitioner and/or the patient such that task compliance can be re-established and enabling data to be identified as valid or discarded and the patient promoted in real-time to continue with the task, thus minimising the amount of invalid data acquired. This is achieved by simultaneously acquiring EEG data which is analysed in real-time and from which it is deduced whether the patient is performing the desired task. The system consists of three parts: (1) fMRI compatible EEG acquisition system, (2) real-time EEG data analysis system, and (3) system providing feedback to the practitioner and/or patient, based on the results of (2) as well as tagging data as valid/invalid if required. Thus, in contrast to fMRI data, EEG data can be easily analyzed in real-time, which can be used to provide real-time feedback to ensure task compliance and also tag in real-time that data is valid/invalid. For example, consider a task-based fMRI scenario in which the patient is asked to watch a video. From the EEG data, one can deduce whether the patient is visually attentive by, e.g. determining the alpha power at the visual cortex. If the patient stops watching the video or switches focus to, e.g., auditory stimuli, feedback is provided directly to the patient or to the practitioner who can remind the patient to watch the video and at the same time the fMRI data acquired can be tagged or identified as being associated with a patient during a time of non-compliance, with the corollary being that data can also be tagged or identified for time periods when the patient is in compliance. In contrast to measuring task compliance with a subsequent questionnaire, EEG based measures are much more objective and can be used instantaneously. Furthermore, the practitioner can adjust the scanning protocol accordingly (e.g. prolong the session, repeat the task etc.) or it could be done automatically. In another embodiment, the EEG can be used for measuring wakefulness of the patient to ensure the patient is either awake or asleep, depending on the needs of the practitioner.

The utilization of EEG data for fMRI has been considered, but in completely different manners to developed here, where the following gives a brief overview of these different utilizations of EEG and fMRI.

C. Zich et al., "Real-time EEG feedback during simultaneous EEG-fMRI identifies the cortical signature of motor imagery", NeuroImage 114 (2015) 438. Describes that motor imagery (MI) combined with real-time electroencephalogram (EEG) feedback is a popular approach for steering brain-computer interfaces (BCI). It is described that MI BCI has been considered promising as add-on therapy to support motor recovery after stroke, and yet whether EEG neurofeedback indeed targets specific sensorimotor activation patterns cannot be unambiguously inferred from EEG alone. In the paper MI EEG neurofeedback is combined with concurrent and continuous functional magnetic resonance imaging (fMRI) to characterize the relationship between MI EEG neurofeedback and activation in cortical sensorimotor areas. EEG signals were corrected online from interfering MRI gradient and ballistocardiogram artifacts, enabling the delivery of real-time EEG feedback. Significantly enhanced task-specific brain activity during feedback compared to no feedback blocks was present in EEG and fMRI. Moreover, the contralateral MI related decrease in EEG sensorimotor rhythm amplitude correlated inversely with fMRI activation in the contralateral sensorimotor areas, whereas a lateralized fMRI pattern did not necessarily go along with a lateralized EEG pattern. Together, it is described that the findings indicate a complex relationship between MI EEG signals and sensorimotor cortical activity, whereby both are similarly modulated by EEG neurofeedback. It is described that this finding supports the potential of MI EEG neurofeedback for motor rehabilitation and helps to better understand individual differences in MI BCI performance. Thus, in the paper by Zich et al., MI EEG neurofeedback is combined with concurrent fMRI to characterise the relationship between MI EEG neurofeedback and activation in cortical sensorimotor areas. The EEG signal is corrected from interfering MRI gradient and ballistocardiogram artifacts in near real-time. EEG data were recorded inside the MRI scanner and validated by comparing the ERD characteristics to separate EEG recordings made outside the scanner from the same participants. A systematic correlation between EEG SMR amplitude reduction and fMRI activity in sense or in motor areas was hypothesised. In other words, the paper describes that EEG raw data is corrected for MRI artefacts.

S. Hoffmann et al., "Crosslinking EEG time-frequency decomposition and fMRI in error monitoring", Brain Struct. Funct. (2014) 219 595-605, describes that studies implicate a common response monitoring system, being active during erroneous and correct responses. It is described that converging evidence from time-frequency decompositions of the response-related ERP revealed that evoked theta activity at fronto-central electrode positions differentiates correct from erroneous responses in simple tasks, but also in more complex tasks. It is described that up to now it is unclear how different electrophysiological parameters of error processing, especially at the level of neural oscillations are related, or predictive for BOLD signal changes reflecting error processing at a functional-neuroanatomical level. The paper aims to provide crosslinks between time domain information, time-frequency information, MRI BOLD signal and behavioral parameters in a task examining error monitoring due to mistakes in a mental rotation task. The results show that BOLD signal changes reflecting error processing on a functional-neuroanatomical level are best predicted by evoked oscillations in the theta frequency band. Although the fMRI results in this study account for an involvement of the anterior cingulate cortex, middle frontal gyrus, and the Insula in error processing, the correlation of evoked oscillations and BOLD signal was restricted to a coupling of evoked theta and anterior cingulate cortex BOLD activity. The current results indicate that although there is a distributed functional-neuroanatomical network mediating error processing, only distinct parts of this network seem to modulate electrophysiological properties of error monitoring. Thus, the paper describes that EEG/fMRI data can be combined in order to establish if certain data can be related to EEG.

WO2013/157012A1 describes a method and a system for monitoring and training attention allocation by applying at least one sensory stimulus over a human subject, using at least one stimulation device, where the sensory stimuli is associated with at least one attentional bias; measuring at least one attention allocation index of the subject by measuring response of the subject to the respective applied sensory stimulus; and outputting an attentional feedback indicative of the measured attention allocation indices. The feedback is outputted in real time or near real time, using one or more output devices for outputting the attention allocation feedback such as visual or auditory output devices. In summary, a patient is stimulated and a response to the stimulation is measured using a measuring device that can be one of: MRI, EEG/ERP etc.

CN102293647B describes a feedback system combining electroencephalogram and functional magnetic resonance signals, which jointly collects and analyzes electroencephalogram and functional magnetic resonance signals, extracts spatial-temporal characteristics of brain specific activity states reflected by the two signals simultaneously, and applies a multi-mode signal to nervous feedback regulation.

US2013/267827A1 describes that in a method and a magnetic resonance (MR) system for functional MR imaging of a predetermined volume segment of the brain of a living examination subject, MR data of the predetermined volume segment are acquired, EEG data of the examination subject are acquired with the acquisition of the EEG data taking place simultaneously with the acquisition of the MR data, and the MR data automatically evaluated dependent on the acquired EEG data. The EEG data is partitioned into frequency classes and the MR data associated with a specific class can then be used to select specific MRI data. Thus, EEG data over a time period is portioned into different frequency data sets, and this is then then used to select specific MRI data.

However, in none of these documents there is a suggestion to utilize EEG data along with task-based information to determine a time window when the patient is, or is not, compliant with a task in order to utilize the associated fMRI data over the same period of time if the patient is in compliance, or disregard and/or prompt the patient if the patient is not in compliance with the task. There is no realisation in any of these documents that EEG can be used in the manner described here in order to determine that a patient is performing the correct task with respect to validation of fMRI data being acquired.

The invention is defined by the appended claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system (10) for functional magnetic resonance image data acquisition, the system comprising:
- an input unit (20);
- a magnetic resonance imaging MRI device (30);
- an electroencephalography EEG data acquisition device (40); and
- a processing unit (50);
wherein, the input unit is configured to provide information relating to one or more tasks to a patient, wherein the information relating to the one or more tasks is provided to the patient over a period of time, wherein the information relating to one or more tasks relates to a visual task and/or an auditory task and/or a motor task and/or a task to enter a resting state and/or a task to fall asleep and/or a task to stay awake;
wherein, the MRI device is configured to acquire functional magnetic resonance imaging fMRI data relating to brain activity of the patient, wherein the fMRI data is acquired over the period of time;
wherein, the EEG device is configured to acquire EEG data relating to electrical activity of the brain of the patient, wherein the EEG data is acquired over the period of time;
wherein, the processing unit is configured to utilize the information relating to the one or more tasks provided over the period of time and the EEG data acquired over the period of time to determine at least one first sub-set period of time over the period of time, wherein the processing unit is configured to analyze the EEG data in real-time to determine if the patient is compliant with at least one task; and
wherein, the processing unit is configured to determine an action associated with acquisition of the fMRI data over the at least one first sub-set period of time, wherein the determined action comprises a provision of a prompt that the patient is not in compliance with at least one task over the at least one first sub-set period of time, wherein the prompt is provided to the patient and/or an operator of the system during the period of time that the patient is not in compliance with at least one task over the at least one first sub-set period of time, and wherein the determined action comprises an identification of the associated fMRI data over the first sub-set period of time as data when the patient was not in compliance with at least one task.

2. System according to claim 1, wherein the processing unit is configured to utilize the information relating to the one or more tasks and the EEG data acquired over the period of time to determine at least one second sub-set period of time over the period of time; and wherein the processing unit is configured to determine an identification of the associated fMRI data over the second sub-set period of time as data when the patient was in compliance with at least one task over the at least one second sub-set period of time.

3. System according to any of claims 1-2, wherein the input unit comprises a visual display unit VDU and wherein provision of information relating to the one or more tasks comprises a display of a video or photograph on the VDU that the patient is requested to watch or look at.

4. System according to claim 3, wherein the utilization of the information relating to the one or more tasks and the EEG data by the processing unit comprises a determination that the patient is watching the video or looking at the photograph.

5. System according to claim 4, wherein the determination that the patient is watching the video or looking at the photograph comprises an analysis of the EEG data to determine a level of visual attentiveness of the patient.

6. System according to any of claims 3-5, wherein the determination that the patient is watching the video or looking at the photograph comprises an analysis of the EEG data to an alpha power at the visual cortex.

7. A method (100) for functional magnetic resonance image data acquisition, the method comprising:
a) providing (110) by an input unit information relating to one or more tasks to a patient, wherein the information relating to the one or more tasks is provided to the patient over a period of time, wherein the information relating to one or more tasks relates to a visual task and/or an auditory task and/or a motor task and/or a task to enter a resting state and/or a task to fall asleep and/or a task to stay awake;
b) acquiring (120) by a magnetic resonance imaging MRI device functional magnetic resonance imaging fMRI data relating to brain activity of the patient, wherein the fMRI data is acquired over the period of time;
c) acquiring (130) by an electroencephalography EEG device EEG data relating to electrical activity of the brain of the patient, wherein the EEG data is acquired over the period of time;
d) utilizing (140) by a processing unit the information relating to the one or more tasks and the EEG data and determining at least one first sub-set period of time over the period of time, and analyzing the EEG data in real-time to determine if the patient is compliant with at least one task; and
f) determining (150) by the processing unit an action associated with acquisition of the fMRI data over the at least one first sub-set period of time, wherein step f) comprises providing a prompt that the patient is not in compliance with at least one task over the at least one first sub-set period of time, wherein step f) comprises providing the prompt to the patient and/or an operator of the system during the period of time that the patient is not in compliance with at least one task over the at least one first sub-set period of time, and wherein step f) comprises identifying the associated fMRI data over the first sub-set period of time as data when the patient was not in compliance with at least one task.

8. Method according to claim 7, wherein the method comprises step e) utilizing (160) by the processing unit the information relating to the one or more tasks and the EEG data acquired over the period of time to determine at least one second sub-set period of time over the period of time; and the method comprises step g) identifying (170) by the processing unit the associated fMRI data over the second sub-set period of time as data when the patient was in compliance at least one task over the at least one second sub-set period of time.

## Patentansprüche

1. System (10) zur funktionellen Magnetresonanz-Bilddatenerfassung, wobei das System umfasst:
- eine Eingabeeinheit (20);
- eine Magnetresonanztomographievorrichtung, MRT-Vorrichtung, (30);
- eine Vorrichtung zum Erfassen von Elektroenzephalographie-, EEG-, Daten (40); und
- eine Verarbeitungseinheit (50);
wobei die Eingabeeinheit dazu konfiguriert ist, einem Patienten Informationen, die sich auf eine oder mehrere Aufgaben beziehen, bereitzustellen, wobei die Informationen, die sich auf die eine oder mehreren Aufgaben beziehen, dem Patienten über einen Zeitraum bereitgestellt werden, wobei sich die Informationen, die sich auf eine oder mehrere Aufgaben beziehen, auf eine visuelle Aufgabe und/oder eine auditive Aufgabe und/oder eine motorische Aufgabe und/oder eine Aufgabe, in einen Ruhezustand zu gelangen und/oder eine Aufgabe zum Einschlafen und/oder eine Aufgabe, wach zu bleiben, beziehen;
wobei die MRT-Vorrichtung dazu konfiguriert ist, funktionelle Magnetresonanztomographie-, fMRT-, Daten, die sich auf Gehirnaktivität des Patienten beziehen, zu erfassen, wobei die fMRT-Daten über den Zeitraum erfasst werden;
wobei die EEG-Vorrichtung dazu konfiguriert ist, EEG-Daten, die sich auf elektrische Aktivität des Gehirns des Patienten beziehen, zu erfassen, wobei die EEG-Daten über einen bestimmten Zeitraum erfasst werden;
wobei die Verarbeitungseinheit dazu konfiguriert ist, die Informationen, die sich auf die eine oder mehreren Aufgaben beziehen, die über den Zeitraum bereitgestellt werden, und die über den Zeitraum erfassten EEG-Daten zu nutzen, um mindestens einen ersten Teilzeitraum über den Zeitraum zu bestimmen, wobei die Verarbeitungseinheit dazu konfiguriert ist, die EEG-Daten in Echtzeit zu analysieren, um zu bestimmen, ob der Patient mindestens eine Aufgabe erfüllt; und
wobei die Verarbeitungseinheit dazu konfiguriert ist, eine Aktion zu bestimmen, die mit Erfassung der fMRT-Daten über den mindestens einen ersten Teilzeitraum verbunden ist, wobei die bestimmte Aktion eine Bereitstellung einer Meldung umfasst, dass der Patient mindestens eine Aufgabe über den mindestens einen ersten Teilzeitraum nicht erfüllt, wobei die Meldung dem Patienten und/oder einem Bediener des Systems während des Zeitraums bereitgestellt wird, in dem der Patient mindestens eine Aufgabe über den mindestens einen ersten Teilzeitraum nicht erfüllt, und wobei die bestimmte Aktion eine Identifizierung der zugehörigen fMRT-Daten über den ersten Teilzeitraum als Daten umfasst, wann der Patient mindestens eine Aufgabe nicht erfüllt hat.

2. System nach Anspruch 1, wobei die Verarbeitungseinheit dazu konfiguriert ist, die Informationen, die sich auf die eine oder mehreren Aufgaben beziehen, und die über den Zeitraum erfassten EEG-Daten zu nutzen, um mindestens einen zweiten Teilzeitraum über den Zeitraum zu bestimmen; und wobei die Verarbeitungseinheit dazu konfiguriert ist, eine Identifizierung der zugehörigen fMRT-Daten über den zweiten Teilzeitraum als Daten zu bestimmen, wann der Patient über den mindestens einen zweiten Teilzeitraum mindestens eine Aufgabe erfüllt hat.

3. System nach einem der Ansprüche 1-2, wobei die Eingabeeinheit eine visuelle Anzeigeeinheit, VDU, umfasst und wobei Bereitstellung von Informationen, die sich auf die eine oder mehreren Aufgaben beziehen, eine Anzeige eines Videos oder Fotos auf der VDU umfasst, das der Patient ansehen oder betrachten soll.

4. System nach Anspruch 3, wobei die Nutzung der Informationen, die sich auf die eine oder mehreren Aufgaben beziehen, und der EEG-Daten durch die Verarbeitungseinheit eine Bestimmung umfasst, dass der Patient das Video ansieht oder das Foto betrachtet.

5. System nach Anspruch 4, wobei die Bestimmung, dass der Patient das Video ansieht oder das Foto betrachtet, eine Analyse der EEG-Daten umfasst, um einen Grad der visuellen Aufmerksamkeit des Patienten zu bestimmen.

6. System nach einem der Ansprüche 3-5, wobei die Bestimmung, dass der Patient das Video ansieht oder das Foto betrachtet, eine Analyse der EEG-Daten auf eine Alpha-Potenz im visuellen Kortex umfasst.

7. Verfahren (100) zur funktionellen Magnetresonanz-Bilddatenerfassung, wobei das Verfahren umfasst:
a) Bereitstellen (110) von Informationen, die sich auf eine oder mehrere Aufgaben beziehen, durch eine Eingabeeinheit für einen Patienten, wobei die Informationen, die sich auf die eine oder mehreren Aufgaben beziehen, dem Patienten über einen Zeitraum bereitgestellt werden, wobei die Informationen, die sich auf eine oder mehrere Aufgaben beziehen, sich auf eine visuelle Aufgabe und/oder eine auditive Aufgabe und/oder eine motorische Aufgabe und/oder eine Aufgabe, in einen Ruhezustand zu gelangen und/oder eine Aufgabe zum Einschlafen und/oder eine Aufgabe, wach zu bleiben, beziehen;
b) Erfassen (120) von funktionellen Magnetresonanztomographie-, fMRT-, Daten, die sich auf Gehirnaktivität des Patienten beziehen, durch eine Magnetresonanztomographie-, MRT-, Vorrichtung, wobei die fMRT-Daten über einen bestimmten Zeitraum erfasst werden;
c) Erfassen (130) von EEG-Daten, die sich auf elektrische Aktivität des Gehirns des Patienten beziehen, durch eine Elektroenzephalographie-, EEG-, Vorrichtung, wobei die EEG-Daten über einen bestimmten Zeitraum erfasst werden;
d) Verwenden (140) der Informationen, die sich auf die eine oder mehreren Aufgaben beziehen, und der EEG-Daten durch eine Verarbeitungseinheit und Bestimmen mindestens eines ersten Teilzeitraums über den Zeitraum und Analysieren der EEG-Daten in Echtzeit, um zu bestimmen, ob der Patient mindestens eine Aufgabe erfüllt; und
f) Bestimmen (150) einer Aktion durch die Verarbeitungseinheit im Zusammenhang mit Erfassung der fMRT-Daten über den mindestens einen ersten Teilzeitraum, wobei Schritt f) Bereitstellen einer Meldung umfasst, dass der Patient mindestens eine Aufgabe über den mindestens einen ersten Teilzeitraum nicht erfüllt, wobei Schritt f) Bereitstellen der Meldung an den Patienten und/oder einen Bediener des Systems während des Zeitraums umfasst, in dem der Patient mindestens eine Aufgabe über den mindestens einen ersten Teilzeitraum nicht erfüllt, und wobei Schritt f) Identifizieren der zugehörigen fMRT-Daten über den ersten Teilzeitraum als Daten umfasst, wann der Patient mindestens eine Aufgabe nicht erfüllt hat.

8. Verfahren nach Anspruch 7, wobei das Verfahren Schritt e) Nutzen (160) der Informationen, die sich auf die eine oder mehreren Aufgaben beziehen, und der über den Zeitraum erfassten EEG-Daten durch die Verarbeitungseinheit umfasst, um mindestens einen zweiten Teilzeitraum über den Zeitraum zu bestimmen; und das Verfahren Schritt g) Identifizieren (170) der zugehörigen fMRT-Daten über den zweiten Teilzeitraum durch die Verarbeitungseinheit als Daten umfasst, wann der Patient mindestens eine Aufgabe über den mindestens einen zweiten Teilzeitraum erfüllt hat.

## Revendications

1. Système (10) d'acquisition de données d'image par résonance magnétique fonctionnelle, le système comprenant :
- une unité d'entrée (20) ;
- un dispositif d'imagerie par résonance magnétique, IRM (30) ;
- un dispositif d'acquisition de données d'électroencéphalographie, EEG (40) ; et
- une unité de traitement (50) ;
dans lequel l'unité d'entrée est configurée pour fournir des informations relatives à une ou plusieurs tâches à un patient, dans lequel les informations relatives à les une ou plusieurs tâches sont fournies au patient sur une période de temps, dans lequel les informations relatives à une ou plusieurs tâches concernent une tâche visuelle et/ou une tâche auditive et/ou une tâche motrice et/ou une tâche pour entrer dans un état de repos et/ou une tâche pour s'endormir et/ou une tâche pour rester éveillé ;
dans lequel le dispositif IRM est configuré pour acquérir des données d'imagerie par résonance magnétique fonctionnelle, IRMf, relatives à une activité cérébrale du patient, dans lequel les données IRMf sont acquises sur la période de temps ;
dans lequel le dispositif EEG est configuré pour acquérir des données EEG relatives à une activité électrique du cerveau du patient, dans lequel les données EEG sont acquises sur la période de temps ;
dans lequel, l'unité de traitement est configurée pour utiliser les informations relatives à les une ou plusieurs tâches fournies sur la période de temps et les données EEG acquises sur la période de temps pour déterminer au moins une première période de temps de sous-ensemble sur la période de temps, dans lequel l'unité de traitement est configurée pour analyser les données EEG en temps réel pour déterminer si le patient est conforme à au moins une tâche ; et
dans lequel l'unité de traitement est configurée pour déterminer une action associée à l'acquisition des données IRMf sur la au moins une première période de temps de sous-ensemble, dans lequel l'action déterminée comprend une fourniture d'une invite indiquant que le patient n'est pas en conformité avec au moins une tâche sur la au moins une première période de sous-ensemble, dans lequel l'invite est fournie au patient et/ou un opérateur du système pendant la période de temps pendant laquelle le patient n'est pas en conformité avec au moins une tâche sur les une ou plusieurs premières périodes de temps de sous-ensemble, et dans lequel l'action déterminée comprend une identification des données IRMf associées sur la première période de temps de sous-ensemble en tant que données lorsque le patient n'était pas en conformité avec au moins une tâche.

2. Système selon la revendication 1, dans lequel l'unité de traitement est configurée pour utiliser les informations relatives à les une ou plusieurs tâches et les données EEG acquises sur la période de temps pour déterminer au moins une seconde période de temps de sous-ensemble sur la période de temps ; et dans lequel l'unité de traitement est configurée pour déterminer une identification des données IRMf associées sur la seconde période de temps de sous-ensemble en tant que données lorsque le patient était en conformité avec au moins une tâche sur les une ou plusieurs seconde périodes de temps de sous-ensemble.

3. Système selon l'une quelconque des revendications 1-2, dans lequel l'unité d'entrée comprend une unité d'affichage visuel VDU et dans lequel une fourniture d'informations relatives à les une ou plusieurs tâches comprend un affichage d'une vidéo ou d'une photographie sur la VDU que le patient est invité à regarder ou à consulter.

4. Système selon la revendication 3, dans lequel l'utilisation des informations relatives à les une ou plusieurs tâches et aux données EEG par l'unité de traitement comprend une détermination selon laquelle le patient regarde la vidéo ou regarde la photographie.

5. Système selon la revendication 4, dans lequel la détermination que le patient regarde la vidéo ou regarde la photographie comprend une analyse des données EEG pour déterminer un niveau d'attention visuelle du patient.

6. Système selon l'une quelconque des revendications 3-5, dans lequel la détermination que le patient regarde la vidéo ou regarde la photographie comprend une analyse des données EEG à une puissance alpha au niveau du cortex visuel.

7. Procédé (100) d'acquisition de données d'image par résonance magnétique fonctionnelle, le procédé comprenant les étapes comprenant :
a) la fourniture (110), par l'intermédiaire d'une unité d'entrée, d'informations relatives à une ou plusieurs tâches à un patient, dans lequel les informations relatives à les une ou plusieurs tâches sont fournies au patient sur une période de temps, dans lequel les informations relatives à une ou plusieurs tâches concernent une tâche visuelle et/ou une tâche auditive et/ou une tâche motrice et/ou une tâche pour entrer dans un état de repos et/ou une tâche pour s'endormir et/ou une tâche pour rester éveillé ;
b) l'acquisition (120), par l'intermédiaire d'un dispositif d'imagerie par résonance magnétique IRM, de données d'imagerie par résonance magnétique fonctionnelle, IRMf, relatives à une activité cérébrale du patient, dans lequel les données IRMf sont acquises sur la période de temps ;
c) l'acquisition (130), par l'intermédiaire d'un dispositif d'électroencéphalographie EEG, de données EEG relatives à une activité électrique du cerveau du patient, dans lequel les données EEG sont acquises sur la période de temps ;
d) l'utilisation (140), par l'intermédiaire d'une unité de traitement, des informations relatives à les une ou plusieurs tâches et des données EEG et la détermination au moins une première période de temps de sous-ensemble sur la période de temps, et l'analyse des données EEG en temps réel pour déterminer si le patient est conforme à au moins une tâche ; et
f) la détermination (150), par l'intermédiaire de l'unité de traitement, d'une action associée à l'acquisition des données IRMf sur au moins une première période de temps de sous-ensemble, dans lequel l'étape f) comprend une fourniture d'une invite indiquant que le patient n'est pas en conformité avec au moins une tâche sur au moins une première période de temps de sous-ensemble, dans lequel l'étape f) comprend une fourniture de l'invite au patient et/ou un opérateur du système pendant la période de temps pendant laquelle le patient n'est pas en conformité avec au moins une tâche sur la au moins une première période de temps de sous-ensemble, et dans lequel l'étape f) comprend une identification des données IRMf associées sur la première période de temps de sous-ensemble en tant que données lorsque le patient n'était pas en conformité avec au moins une tâche.

8. Procédé selon la revendication 7, dans lequel le procédé comprend l'étape e) d'utilisation (160), par l'intermédiaire de l'unité de traitement, des informations relatives à les unes ou plusieurs tâches et aux données EEG acquises sur la période de temps pour déterminer au moins une seconde période de temps de sous-ensemble sur la période de temps ; et le procédé comprend l'étape g) d'identification (170), par l'intermédiaire de l'unité de traitement, des données IRMf associées sur la seconde période de temps de sous-ensemble en tant que données lorsque le patient était en conformité avec au moins une tâche sur la au moins une seconde période de temps de sous-ensemble.
